(19) **Europäisches Patentamt**
**European Patent Office**
**Office européen des brevets**

(11) Veröffentlichungsnummer: **0 270 068**
**A2**

(12) # EUROPÄISCHE PATENTANMELDUNG

(21) Anmeldenummer: **87117738.2**

(22) Anmeldetag: **01.12.87**

(51) Int. Cl.⁴: **A61K 37/02** , C12N 5/00 , A23K 1/18

(30) Priorität: **03.12.86 DE 3641265**

(43) Veröffentlichungstag der Anmeldung:
**08.06.88 Patentblatt 88/23**

(84) Benannte Vertragsstaaten:
**AT BE CH DE ES FR GB GR IT LI LU NL SE**

(71) Anmelder: **Bissendorf Peptide GmbH**
**Burgwedeler Strasse 25**
**D-3002 Wedemark 2(DE)**

(72) Erfinder: **Mach, Walter, Dr.**
**Wasserburgerstrasse 17**
**D-8011 München-Kirchseeon(DE)**

(74) Vertreter: **Werner, Hans-Karsten, Dr. et al**
**Deichmannhaus am Hauptbahnhof**
**D-5000 Köln 1(DE)**

(54) **Organspezifische Peptide, ihre Gewinnung und Verwendung.**

(57) Organspezifische Peptide sind die natürlichen Prophylaktika bzw. Arzneimittel bei der Verhütung bzw. der Therapie kataboler Organentgleisungen und die natürlichen Geriatrika. Die organspezifischen Peptide lassen sich aus Tierseren bzw. menschlichen Seren, aber auch aus tierischen und menschlichen Organen gewinnen. Ein tierisches Serum mit erhöhtem Gehalt an organspezifischen Peptiden kann gewonnen werden durch Fütterung von Tieren mindestens 20 Tage vor dem Schlachten mit einem Futter, in dem die Kohlenhydrate zu mehr als 50 % isokalorisch durch Fett ersetzt sind. Das Serum mit erhöhtem Gehalt an organspezifischen Peptiden kann für Gewebekulturen, aber auch zur Herstellung von Konzentraten und Konzentratfraktionen verwendet werden, die in Therapie, Prophylaxe und Geriatrie einsetzbar sind.

EP 0 270 068 A2

## Organspezifische Peptide, ihre Gewinnung und Verwendung

Gegenstand der vorliegenden Erfindung sind zellspezifische anabole Peptide, die beim Einzeller und beim höheren Organismus für die anabole Seite der Stoffwechselregulation eingesetzt werden. Sie lassen sich durch ein besonderes Verfahren im Serum von Tieren anreichern und aus dem Serum gewinnen. In der Therapie und Prophylaxe (Human-und Veterinärmedizin) sind sie für die Normalisierung bzw. Verhütung kataboler Stoffwechselentgleisungen die biologischen Arzneimittel der Wahl. Außerdem lassen sie sich als Geriatrika verwenden.

Jede Zelle benötigt für eine normale Funktion einen intakten Energie-und Synthesestoffwechsel. Bei aerob lebenden Einzellern ist die Energieproduktion vom $O_2$-Partialdruck abhängig, da der Sauerstoff als Elektronenakzeptor für die ATP-bildenden Elektronentransportsysteme eingesetzt wird. Die oxydative Energieproduktion ist aber mit der Bildung von Radikalen und Peroxyden verknüpft, durch die der Synthesestoffwechsel in Mitleidenschaft gezogen wird. Die Synthesen benötigen reduzierendes Milieu. Um einen ungestörten Synthesestoffwechsel parallel zur aeroben Energiegewinnung zu ermöglichen, ist im Rahmen der Evolution schon sehr früh ein sogenanntes "anaboles", die Synthesen gegenüber dieser Radikalbildung abschirmendes Peptid entwickelt worden, das von der jeweiligen Zelle selbst gebildet wird. Der Wachstumsprozeß wird u.a. mit der Induktion dieser Peptidsynthese eingeleitet. Diese "zellspezifischen" Peptide, die während der Wachstumsphase auch an das Nährmedium abgegeben werden, sind beim Einzeller schon vor längerer Zeit entdeckt und zunächst als "Strepogenine" bezeichnet worden (Woolley, "Some Correlations of Growth-Promoting Powers of Proteins with Their Strepogenin Content", Journal of Biological Chemistry, Band 162, 1946, Seiten 383 - 388).

Bei der Weiterentwicklung des Einzellers zum höheren Organismus wurde dieses regulatorische Grundprinzip beibehalten: Jede Zelle und damit jedes Organ bildet selbst ein zell-bzw. organspezifisches Peptid (La Brecque und Bachur, "Hepatic Stimulator Substance: Physicochemical Characteristics and Specificity", American Journal of Physiology, Band 242, 1982, Seiten G281 - G288; La Brecque, "In Vitro Stimulation of Cell Growth by Hepatic Stimulator Substance", American Journal of Physiology, Band 242, 1982, Seiten G289 - G295; Goldstein, Asanuma und White, "The Thymus as an Endocrine Gland: Properties of Thymosin, a New Thymus Hormone", in: Recent Progress in Hormone Research, (Ed. Astwood) Band 26 (1970), Seiten 505 - 538, Academic Press, New York, London; Luft und Hall (Eds.), "Somatomedins and Some Other Growth Factors", in: Advances in Metabolic Disorders, Band 8, 1975, Academic Press, London, New York, San Francisco; Baserga (Ed.), "Tissue Growth Factors", in: Handbook of Experimental Pharmacology, Band 57, 1981, Springer-Verlag, Berlin, Heidelberg, New York), das die Hemmung des Synthesestoffwechsels durch die mit der Energiebereitstellung verbundene Radikalbildung verhindert. Es handelt sich hier also um eine besondere Peptidgruppe, die von den zahlreichen schon bekannten Peptiden, die der funktionellen Organkoordination dienen, wie z.B. die Regulationspeptide des Hypothalamus, der Hypophyse, des Magen-Darmtraktes, des Pankreas, des Nervensystems sowie die für die Regulation des $Ca^{++}$-Spiegels zuständigen Faktoren (Parathormon, Calcitonin), prinzipiell zu trennen ist (Blundell und Humbel, "Hormone Families: Pancreatic Hormones and Homologous Growth Factors", Nature, Band 287, 1980, Seiten 781 787). Allerdings mußte im Rahmen der beim höheren Organismus erforderlichen Organkoordination der $O_2$-Partialdruck konstant gehalten werden, so daß weitere Regulatoren für den Energiestoffwechsel benötigt wurden: Corticosteroide und Schilddrüsenhormone ($T_3$ /$T_4$). Die Basisregulation bei den Zellen des höheren Organismus besteht daher in der Aufrechterhaltung eines stationären Zustandes zwischen der Energieproduktion (Corticosteroide + $T_3$ /$T_4$) und des Synthesestoffwechsels (zellspezifisches, anaboles Peptid). Die Induktion der Peptidsynthese erfolgt bedarfsgerecht durch die "tropen" Hormone der Hypophyse. Auch diese zellspezifischen Peptide werden - wie beim Einzeller - z.T. an das Blut (= "Nährmedium") abgegeben, so daß sie im Serum analytisch erfaßt und aus dem Serum präparativ gewonnen werden können.

Während der Wachstumsphase wird viel Peptid gebildet. Beim ausgewachsenen Organismus ist der Umfang der Peptidsynthese einerseits von der Reproduktionsrate abhängig: Auf Reproduktion angewiesene Gewebebereiche wie Epithelien, Endothelien, Bindegewebe und Zellen des Abwehrsystems weisen auch hier noch eine kontinuierlich hohe Syntheserate auf, während diese bei anderen Organen sehr gering ist. Andererseits erfolgt eine Induktion bei besonderen Belastungen.

Der beschriebene stationäre Zustand zwischen Energie-und Synthesestoffwechsel kann nach beiden Seiten entgleisen: Überwiegt die Energiebildung, so bricht der Synthesestoffwechsel durch überhöhte Radikalbildung zusammen ("katabole Entgleisung"). Ist die Bildung des zellspezifischen Peptids zu hoch, so wird der Energiestoffwechsel durch das Peptid gehemmt ("anabole Entgleisung"). Derartige Störungen, die durch psychische Belastungen, Infektionen oder andere beliebige Noxen eingeleitet werden können, sind als "Regulationskrankheiten" bekannt geworden, die für praktisch jedes Organ bzw. für jeden Gewebebereich existieren.

Anabole Entgleisungen lassen sich mit Corticosteroiden behandeln. Hier liegen schon weitgehende Erfahrungen vor. Die Behandlung kataboler Entgleisungen gelingt beim jüngeren Organismus durch Kohlenhydratentzug. Die Konzentration der zellspezifischen Peptide nimmt - gemessen an der gesamtanabolen Aktivität des Serums - unter diesen Bedingungen sehr stark zu, wie das Beispiel 1 (s.u.) zeigt. Der Effekt erklärt sich durch Ersatz des bei kohlenhydratreicher Ernährung dominierenden Insulins durch das Wachstumshormon (STH), das die Bildung der gewebespezifischen Peptide induziert, die dann auch ans Blut abgegeben werden. Die Resistenz gegen Infektionen (Bakterien; Parasiten), Schadstoffe (Tetrachlorkohlenstoff; Carcinogene) und Strahlung wird signifikant erhöht, die Neigung zu Arteriosklerose vermindert (Langzeitversuch am Huhn) (Schole, Harisch und Sallmann, "Belastung, Ernährung und Resistenz", 1978, Verlag Paul Parey, Hamburg, Berlin; Haas, "Der Einfluß different gestalteter Kohlenhydrat-Fett-Diäten auf die Inzidenz Azoxymethan-induzierter Tumoren bei Ratten", 1984, Dissertation Tierärztliche Hochschule Hannover). Katabole Organentgleisungen werden normalisiert (Beispiele: Kataboles ulcus ventriculi et duodeni/M. Crohn (Magen-und Darmschleimhaut); Herzinsuffizienz/Endocarditis ulcerosa (Herz); Osteoporose/Arthrosen (Binde-und Stützgewebe) (Lutz, "Leben ohne Brot", 9. Auflage, 1985, Selecta-Verlag, Planegg vor München). Beim älteren Organismus gelingt die Normalisierung derartiger kataboler Entgleisungen durch Kohlenhydratrestriktion weniger gut, weil die STH-Produktion in der Hypophyse teilweise nur noch sehr schwer aktiviert werden kann. Hier ist eine kausale Therapie nur durch Zuführung der jeweiligen gewebespezifischen Peptide möglich. Da sie nach allen bisherigen Indizien organspezifisch und nicht artspezifisch sind, können die Peptide des nach besonderem Verfahren gewonnenen Schweine-Serums (Beispiel 1), aber auch eines embryonalen Kälberserums für die Therapie eingesetzt werden. Da es aber nicht sicher ist, daß die Aminosäurensequenzen absolut mit den entsprechenden Peptiden des Menschen übereinstimmen, trotz etwa gleicher biologischer Aktivität, ist die Isolierung, Sequenzierung und Synthese der gewebespezifischen Peptide des Menschen anzustreben. Sie lassen sich ganz analog aus menschlichem Serum gewinnen.

Serum mit einem erhöhten Gehalt an organspezifischen Peptiden ist bisher in erheblichem Umfang für Gewebekulturen verwendet worden. Hierzu wurde vor allem kalbsfötales Serum eingesetzt, das in nur relativ geringen Mengen und zu sehr hohen Preisen erhältlich ist.

Die Erfindung hat sich zunächst die Aufgabe gestellt, ein anderes Serum mit erhöhtem Gehalt an organspezifischen Peptiden zur Verfügung zu stellen, welches preiswerter und in größeren Mengen zur Verfügung gestellt werden könnte. In größeren Mengen stehen eigentlich nur die Seren von Schlachtvieh, insbesondere Schweinen und Rindern zur Verfügung. Diese Seren enthalten jedoch im allgemeinen nur sehr geringe Mengen an organspezifischen Peptiden, so daß die Anreicherung oder Gewinnung von organspezifischen Peptiden hieraus bisher als völlig unwirtschaftlich nicht in Betracht gezogen wurde.

Im Rahmen von eingehenden Untersuchungen über die physiologische Auswirkung verschiedenartig zusammengesetzter Futtermischungen wurde festgestellt, daß es möglich ist, bei Schweinen innerhalb von 10 bis 20 Tagen den gesamten Metabolismus umzustellen, sofern man sie mit einer kohlenhydratarmen und isokalorisch fettreichen Futtermischung füttert. Diese Umstellung des Metabolismus führt zu einem Abbau des Fettes und einer verstärkten Bildung von Fleisch. Weiterhin wurde festgestellt, daß diese Umstellung des Metabolismus mit einem sprunghaften Anstieg des Serums an organspezifischen Peptiden verbunden ist.

Der verstärkte Fleischansatz und die bessere Ausbildung der sonstigen Organe unter gleichzeitigem Abbau der Fettreserven wird demnach bewirkt durch eine erhöhte Produktion und Ausschüttung organspezifischer Peptide mit stark anaboler Wirkung. Diese Umstellung des Metabolismus zeigt sich bereits nach ca. 20 Tagen bei Verwendung eines Futters, in dem die Kohlenhydrate zu mehr als 50% isokalorisch durch Fett ersetzt sind. Hierbei steigt der Gehalt an organspezifischen Peptiden schon etwa um den Faktor 3.

Bei Verwendung eines Futters, in dem die Kohlenhydrate zu mehr als 90% isokalorisch durch Fett ersetzt sind, finden sich nach mindestens 30 Tagen Konzentrationen an organspezifischen Peptiden, die um den Faktor 50 über dem normalen Serumspiegel liegen.

Erfindungsgemäß ist es somit möglich, durch eine spezielle Fütterung in der Zeit vor der Schlachtung nicht nur fettarme und fleischreiche Schlachtschweine, sondern auch ein Schweineserum mit erhöhtem Gehalt an organspezifischen Peptiden zu erzeugen.

Da die isokalorische Menge an Fett im Vergleich zu Kohlenhydraten erheblich niedriger ist, muß dem Futter ein inerter Ballaststoff zugegeben werden, der im Verdauungstrakt des Schweines nicht zu Kohlenhydraten gespalten wird. Als inerter Ballaststoff hat sich Polyethylenpulver hervorragend bewährt. Prinzipiell sind aber auch andere geschmacksneutrale, inerte Ballaststoffe geeignet, die von den Schweinen als Futter akzeptiert werden und die nicht zu Kohlenhydraten gespalten werden.

Die erfindungsgemäß brauchbaren Futtermischungen enthalten darüber hinaus die üblichen Komponenten wie Vitamine, Mineralstoffe sowie Protein, beispielsweise in Form von Weizenkleie, Sojaschrot und Fischmehl.

Als Fette können alle in ausreichendem Maße vorhandenen Fette eingesetzt werden wie Schweineschmalz, Rindertalg und/oder Pflanzenöle.

Die Gewinnung des Serums erfolgt in an sich bekannter Weise bei der Schlachtung durch Gewinnung der Serumfraktion aus dem Blut.

Erfindungsgemäß wird es in Zukunft möglich sein, den Bedarf an Serum mit hohem Gehalt an organspezifischen Peptiden nicht nur aus Kalbsföten zu decken, sondern aus Schweinen, die vier bis sechs Wochen vor der Schlachtung auf ein Futter umgestellt worden sind, in dem die Kohlenhydrate zu mehr als 50%, vorzugsweise mehr als 90% isokalorisch durch Fett ersetzt sind. Sobald von derartigem Serum ausreichende Mengen zur Verfügung stehen, wird es weiterhin möglich sein, Konzentrate und/oder Konzentratfraktionen organspezifischer Peptide herzustellen und sie in der Therapie, Prophylaxe und Geriatrie einzusetzen. Dies ist insbesondere dadurch möglich, daß sich inzwischen herausgestellt hat, daß die organspezifischen Peptide nicht artspezifisch sind. Selbst wenn sich geringfügige Abweichungen in der Peptidstruktur finden sollten, so sind diese Peptide dennoch bei den verschiedensten Arten wirksam. Das erfindungsgemäße Verfahren zur Herstellung von Schweineserum mit erhöhtem Gehalt an organspezifischen Peptiden eröffnet somit völlig neue Möglichkeiten der Verwendung dieser organspezifischen Peptide mit stark anaboler Aktivität.

In den nachfolgenden Beispielen wird die Erzeugung des Schweineserums sowie die Anreicherung der Peptide im Blut in Abhängigkeit von der Fütterungszeit näher erläutert:

BEISPIEL 1:

Eine geeignete Futtermischung für Schweine, in der Kohlenhydrate weitgehend isokalorisch ersetzt sind durch Fette, hat folgende Zusammensetzung:

| Futterkomponente | % |
|---|---|
| Weizenkleie | 2,0 |
| Sojaschrot | 12,0 |
| Fischmehl | 16,0 |
| Sojaöl | 0,25 |
| Vitamine [1] | 0,25 |
| Mineralstoffe [2] | 2,0 |
| Fettmischung (Talg/Schmalz 1:1) | 30,0 |
| Polyethylenpulver (Hostalen®) | 37,5 |
| | 100,0 |

[1] 1 kg Futter enthält folgende Vitaminanteile:

| Vitamin A: | 6000 IE | Vitamin $B_6$: | 2,25 mg |
|---|---|---|---|
| $D_3$: | 750 IE | $B_{12}$: | 12 µg |
| E: | 9 mg | $K_3$: | 0,75 mg |
| $B_1$: | 0,75 mg | Cholinchlorid: | 200 mg |
| $B_2$: | 3 mg | Folsäure: | 6 mg |
| Ca-D-Pantothenat: | 4,5 mg | Biotin: | 0,6 mg |
| Nicotinsäure: | 12 mg | Inositol: | 75 mg |
| | | Vitamin C: | 60 mg |

ad 2,5 g Weizennachmehl

[2] 1 kg Futter enthält folgende Mineralstoffanteile:

| Kohlensäure-Futterkalk: | 10,8 g |
|---|---|
| Phosphorsäure-Futterkalk: | 6 g |
| Viehsalz: | 3 g |
| Spurenelemente (als Sulfate; Fe:Zn:Mn:Cu = 5:8:2:1): | 0,2 g |

Sechs Schweine wurden mit einem handelsüblichen Schweinemast-Fertigfutter bis zu einem Gewicht von 70 bis 75 kg gefüttert und dann für 28 Tage auf das oben genannte Fettfutter umgestellt. Die Serumgewinnung erfolgte bei der Schlachtung in üblicher Weise. Anschließend wurde einerseits die gesamtanabole Aktivität des Serums mit Hilfe des sogenannten Glutathion-Statustestes ermittelt (Schole, Harisch und Sallmann, "Belastung, Ernährung und Resistenz", Beiheft Nr. 9 zur Zeitschrift für Tierphysiologie, Tierernährung und Futtermittelkunde, 1978, Seite 11; Schole, "Theory of Metabolic Regulation Including Hormonal Effects on the Molecular Level", Journal of Theoretical Biology", Band 96, 1982, Seiten 579 - 615, sowie Schole, Grönert und Eikemeyer, "Untersuchungen über die direkte Wirkung von Wachstumsförderern auf den Synthesestoffwechsel der Leber", Zeitschrift für Tierphysiologie, Tierernährung und Futtermittelkunde, Band 54, 1985, Seiten 27 bis 41). Hierbei werden membranständige Flavinenzyme in vivo gehemmt. Die Abnahme der Radikal-und Peroxidbildung führt sehr schnell zu einer Reduktion von Glutathion-S-Radikalen bzw. von gemischten Glutathion-Disulfiden, so daß der GSH-Anstieg als Maß für die anabole Aktivität einer Verbindung verwendet werden kann. Die zu testenden Substanzen werden intraportal bei Ratten injiziert und das SH-Glutathion nach 1 bis 10 Minuten, in der Regel nach 2 Minuten gemessen.

In dem Zeitraum von 10 Minuten ändert sich die Konzentration an Gesamtglutathion nicht, so daß ausschließlich Redoxverschiebungen erfaßt werden. Außerdem besteht die Gewähr, daß weder Enzyminduktionen noch Darmfloraeffekte die Meßergebnisse verfälschen. Diese Methode gestattet, die gesamtanabole Aktivität des Serums zu bestimmen.

Die folgende Tabelle 1 enthält die Ergebnisse im Vergleich mit handelsüblichen Serumproben. Als Bezug wurde die anabole Aktivität eines Casein-Hydrolysates verwendet. (1 Caseinpeptid-Einheit = 1 μg eines Caseinpeptidgemisches, das durch tryptische Spaltung von Casein und anschließender Reinigung gewonnen worden ist.)

## Tabelle 1

| Art des Serums | Caseinpeptideinheiten (pro ml) |
|---|---|
| Rinderserum | 80 |
| Kälberserum | 400 |
| Fötales Kälberserum | 666 |
| Schweineserum (28 Tage Fettfutter) | 5714 |

Die "gesamtanabole Aktivität" des Schweineserums liegt demnach 28 Tage nach der Futterumstellung 8,6-fach über der des handelsüblichen fötalen Kälberserums.

Der Verlauf der Peptidanreicherung im Serum nach Verfütterung der Fettdiät wurde im Rattenversuch ermittelt. Es wurde die folgende halbsynthetische Diät verwendet, in der reine Maisstärke gegen reines Fett (Talg/Schmalz 1:1) isoenergetisch ausgetauscht werden konnte:

Zusammensetzung der Diäten in %:

| Futterkomponente | Kohlenhydrat-reiche Diät | Fettdiät 30 % |
|---|---|---|
| Assay-Sojaprotein | 10,0 | 10,0 |
| Magermilchpulver | 10,0 | 10,0 |
| Maisstärke | 68,4 | – |
| Fett (Schmalz/Talg 1:1) | – | 30,0 |
| Methionin | 1,0 | 1,0 |
| Sojaöl | 1,0 | 1,0 |
| Mineralstoffmischung [1] | 3,0 | 3,0 |
| Vitaminmischung [2] | 1,0 | 1,0 |
| Hostalenmehl | 5,6 | 44,0 |
| | 100,0 | 100,0 |

[1]Mineralstoffe pro kg Endmischung in g:

```
8,000  kohlensaurer Kalk
6,000  phosphorsaurer Kalk
4,800  Viehsalz
1,000  MgSO4
0,100  Spurenelemente (Fe:Cu:Mn:Co (als Sulfate)
                        = 5:1,5:1:0,2)
0,050  ZnCO3
0,040  KJ
0,010  NaF
10,000  KCl

30,000
```

[2]Vitamin- und Wirkstoffzusatz pro kg Endmischung:

```
200  mg Vit. E 25 %          50   mg Nikotinsäure
  2  mg Vit.K3                 2   g  Cholinchlorid
 20  mg Vit. B2                2   mg Folsäure
 20  mg Vit. B1 x HCl        200   µg Biotin
 10  mg Vit. B6 x HCl        250   mg Inositol
 30  µg Vit. B12              20   mg Vit. C
 50  mg Ca-Panthotenat
 60  mg Vit. A und D3 (1 g = 400.000 I.E. Vit. A
                             50.000 I.E. Vit. D3)
ad 10,0 g Casein
```

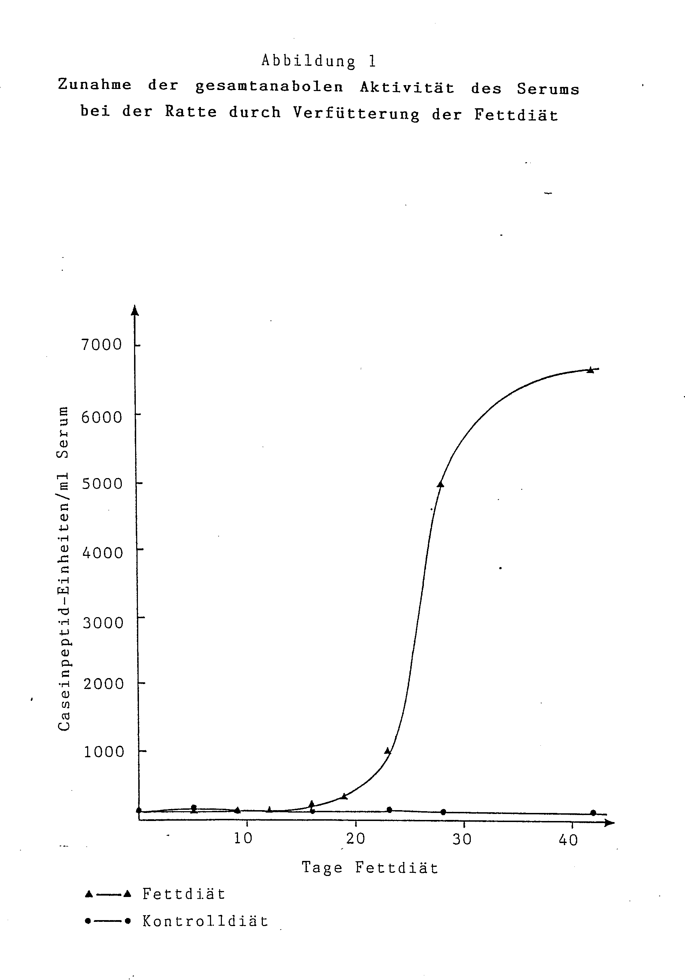

Die Diäten wurden insgesamt 42 Tage verfüttert und nach verschiedenen Zeiten das Serum von je 6 kohlenhydratreich-bzw. fettreich gefütterten Tieren im Glutathionstatustest untersucht. Das Ergebnis zeigt die Abb. 1.

**Beispiel 2**

Die Hauptpeptidfraktion des Schweineserums wurde nach folgender Methode gewonnen:

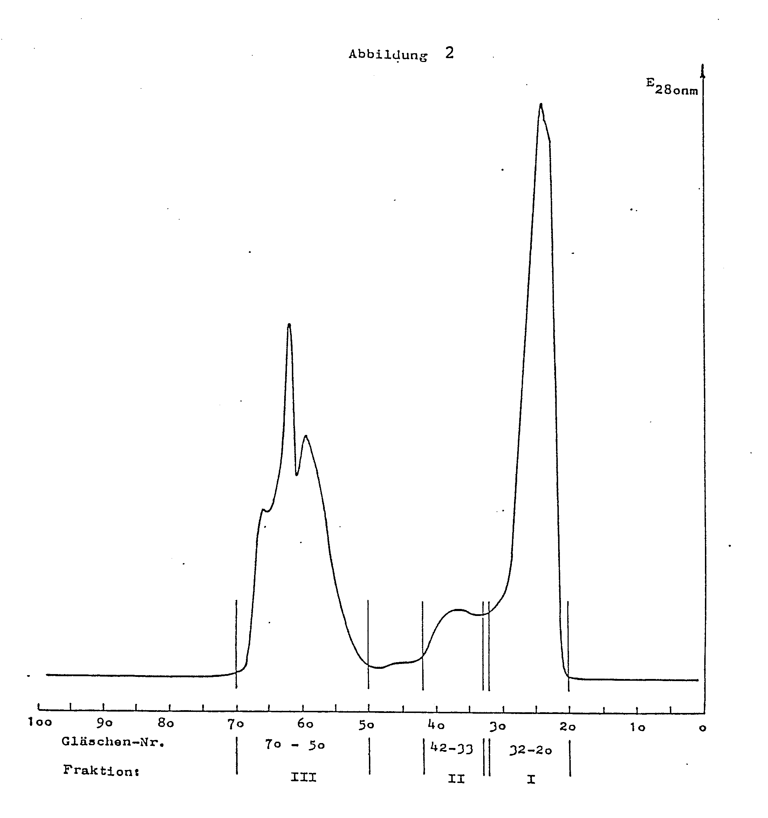

Jeweils 2 ml Serum wurden in einem 10 ml-Zentrifugenglas bei 100°C im Heizblock 10 min denaturiert. Anschließend wurden 5 Gläschen in einem 100 ml Zentrifugenglas vereinigt und 3 mal mit je 5 ml Wasser (bidest.) mit Hilfe eines Ultra-Turrax (Jahnke und Kunkel, Staufen) gewaschen. Nach jedem Waschvorgang wurde zentrifugiert und abschließend wurden alle Überstände vereinigt und lyophilisiert. 500 mg des trockenen Lyophilisates wurden dann in 5 ml bidest. Wasser gelöst und an Sephadex G50 ("fine") chromatographiert (Säulenhöhe: 25 cm, Durchmesser: 2,5 cm; Äquilibrierung und Entwicklung der Säule mit bidest. Wasser). Mit einem Fraktionssammler wurden 50 Tropfen pro Glas bei einer Laufgeschwindigkeit von 100 ml/h aufgefangen. Das Elutionsdiagramm zeigt die Abbildung 2. Die Fraktionen I und II waren frei von NaCl. Die Fraktion III enthielt NaCl und die gesamten niedermolekularen Anteile. Die Testung der Fraktionen I - III im Glutathionstatustest führte zu den in der Tabelle 2 aufgeführten Ergebnissen.

## T a b e l l e 2

| Fraktion | µg | µmol SH-Glutathion pro g Frischleber | | Änderung % | Signifikanz (p zu NaCl) |
|---|---|---|---|---|---|
| | | NaCl | Fraktion | | |
| I + II | 2,5 | 5,33+0,23 | 6,32+0,24 | + 18,6 | < 0,01 |
| I | 2,5 | 5,14+0,25 | 5,84+0,11 | + 13,6 | < 0,01 |
| II | 2,5 | 5,32+0,34 | 5,38+0,21 | + 0 | - |
| III | 2,5 | 5,26+0,41 | 5,30+0,39 | + 0 | - |

(Mittelwerte von sechs Schweinen; Resorptionszeit 2 min).

Demnach enthält die Fraktion I offensichtlich die anabol wirksamen Peptide.

Das Spektrum dieser Fraktion weist die für aromatische Aminosäuren charakteristische Absorptionsbande bei 280 nm auf. Der mit der Biuretmethode und nach Lowry bestimmte Peptidgehalt dieser Fraktion liegt bei 608 mg/g, so daß diese Fraktion 61% Peptid enthält. Pro l Serum lassen sich nach dieser Methode 451 ± 39 mg reines Peptid gewinnen.

**Beispiel 3**

Die Testung des Serums an glatten Muskelzellen aus Rattenaorta in der Gewebekultur führte zu den folgenden Ergebnissen:

Unter sterilen Bedingungen wurden aus Rattenaorten Würfel (ca. 1 mm Kantenlänge) gewonnen und von Adventitia-und Mediagewebe befreit.

Etwa 30 dieser Würfel wurden in 10 ml Dulbecco's Medium (Modifikation DMEM) gebracht.

Die Serumzusätze wurden variiert (siehe unten). Wechsel des Mediums und des Serumzusatzes erfolgten an jedem 3. Tag.

Nach 9 Tagen wurden die ausgewachsenen Zellen in 20 ml desselben Mediums plus Serumzusatz (1. Subkultur; Mediumwechsel jeden 3. Tag) und nach einer weiteren Woche je 50.000 Zellen in je 5 ml Medium plus Serum überführt (2. Subkultur). Das Wachstum der 2. Subkultur wurde über 10 Tage (ohne Mediumwechsel) verfolgt. Die Ergebnisse zeigt die folgende Tabelle 3:

## T a b e l l e 3

Wirkung unterschiedlicher Serumzusätze auf die Proliferation glatter Muskelzellen der Rattenaorta (Angaben in Zellzahl/5 ml Medium; n = 6 Ratten)

| Serum | Zusatz in % | Auszählung nach Tagen | |
|---|---|---|---|
| | | 5 | 10 |
| Fötales Kälberserum | 2 | kein Wachstum | kein Wachstum |
| | 5 | 62.783 + 561 | 108.476 + 2.171 |
| | 10 | 130.125 + 11.243 | 811.367 + 151.761 |
| Schweineserum nach Fett- fütterung | 2 | 143.861 + 13.583 | 921.917 + 101.316 |

Die wachstumsstimulierende Wirkung des Schweineserums liegt 28 Tage nach der Futterumstellung - gemessen an glatten Muskelzellen der Rattenaorta in der Gewebekultur - etwa 5-fach über dem Effekt eines handelsüblichen fötalen Kälberserums.

## Beispiel 4

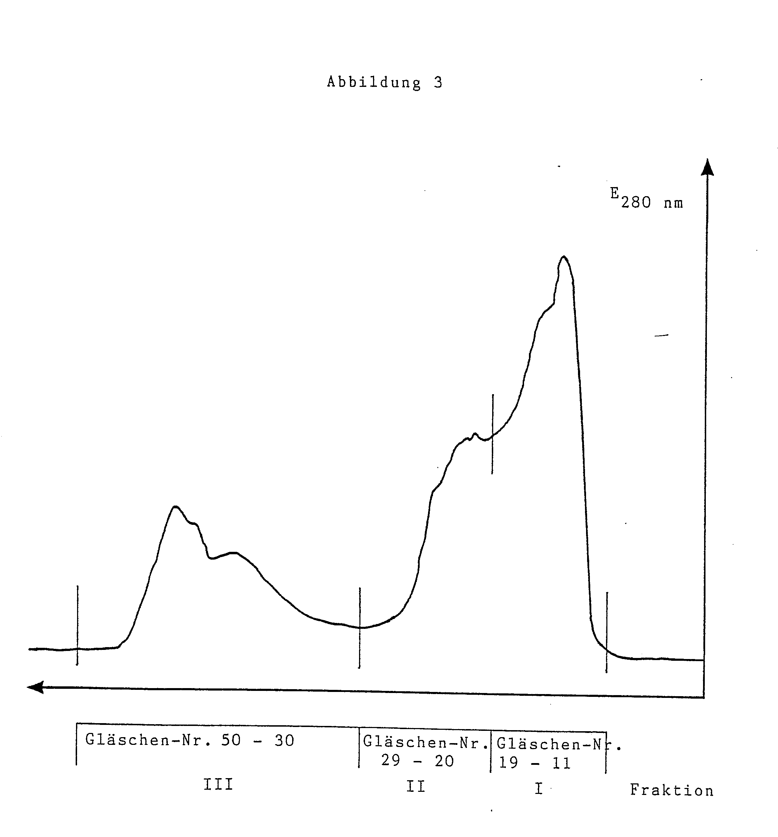

Ganz analog wurde ein handelsübliches fötales Kälberserum aufgearbeitet. In diesem Fall wurden nur 150 mg des trokkenen Lyophilisates für die Sephadexchromatographie verwendet. Das Lyophilisat wurde in KOH (1 mol/l) gelöst und mit $HClO_4$ neutralisiert. Das Elutionsdiagramm ist in der Abb. 3 wiedergegeben. Das Ergebnis des Glutathionstatustestes enthält die Tabelle 4. Der Peak III wurde nicht mehr untersucht. Bei diesem Serum muß vom Peak I etwa die 5-fache Menge eingesetzt werden, um eine signifikante Reduktion des Glutathionsystems zu erhalten. Andererseits ist der Peak II schon im Konzentrationsbereich von 2,5 µg wirksam. Es ergeben sich also deutliche Unterschiede zwischen dem mit der Fettdiät erzeugten Schweineserum und dem fötalen Kälberserum. Man muß daher verschiedene Tierseren, u.a. auch das fötale Kälberserum, zur Gewinnung der gewebespezifischen Peptide für die Verwendung in der Therapie, Prophylaxe und Geriatrie heranziehen. Da die Peptide nach allen bisher greifbaren Indizien organspezifisch und nicht artspezifisch sind, müßte die Wirksamkeit beim Menschen gegeben sein (Schole und Lutz, "Regulationskrankheiten - Versuch einer fachübergreifenden Analyse", 1987, in Vorbereitung).

### T a b e l l e 4

| Fraktion | µg | µmol SH-Glutathion pro g Frischleber | | Änderung % | Signifikanz (p zu NaCl) |
|---|---|---|---|---|---|
| | | NaCl | Fraktion | | |
| I | 12,5 | 5,41 + 0,39 | 6,24 + 0,42 | +15,3 | < 0,01 |
| II | 2,5 | 5,57 + 0,44 | 6,17 + 0,62 | +10,7 | < 0,05 |

(Mittelwerte von sechs Ratten; Resorptionszeit 2 min).

## Beispiel 5

10 männliche Patienten mit einem Ulcus ventriculi wurden mit täglich je 100 mg der aus Schweineserum gewonnenen Peptidfraktion oral behandelt. Nach 4 Wochen war in 9 Fällen das Ulcus klinisch ausgeheilt. Der zehnte Patient gab eine deutliche Verminderung der subjektiven Beschwerden zu Protokoll.

## Beispiel 6

10 Patienten (5 Männer und 5 Frauen) mit M. Crohn, die durch Kohlenhydratrestriktion nicht gebessert werden konnten ("Versager" nach Lutz, "Kohlenhydratarme Diät bei Colitis ulcerosa und Morbus Crohn", Colo-Proctology III, Seiten 349 - 355), wurden 3 Monate mit täglich je 100 mg der Peptidfraktion aus Schweineserum oral behandelt. In dieser Zeit wurden alle Patienten wesentlich gebessert (Auswertung anhand des Aktivitätsindex nach Best (Hotz und Goebell, "Bedeutung von Aktivitätsindices beim Morbus Crohn", Verdauungskrankheiten, Band 1, 1983, Seiten 36 - 39).

## Beispiel 7

Die Peptidfraktion aus Schweineserum wurde separat und zusammen mit Corticosteroiden mit folgenden Zusätzen zu einer Salbe verarbeitet:

Zutaten in g:

| | |
|---|---|
| Walrat | 10 |
| Bienenwachs | 12 |
| Kampfer | 4 |
| Olivenöl | 30 |
| Lebertran | 12,5 |
| Aqua dest. | 30 |
| Benzoesäure | 0,5 |
| Peptide + Cortisol | 1 |
| | 100 |

(enthaltend 1 g Peptidfraktion bzw. 965 mg Peptidfraktion und 35 mg Cortisol)

Hauterkrankungen wie Akne vulgaris konnten mit der reinen Peptidsalbe in relativ kurzer Zeit (2 - 4 Wochen) wesentlich gebessert werden. Entzündungen der Haut und verschiedenste Verletzungen ließen sich mit der Zweikomponenten-Salbe in kürzerer Zeit zur Abheilung bringen, als es mit einer reinen Cortisonsalbe möglich war.

## Beispiel 8

Ein Vergleich der Zusammensetzung der gemäß Beispiel 1 verwendeten geschlachteten Schweine mit der Zusammensetzung von Schweinen, die bis zur Schlachtung mit dem üblichen Futter weitergefüttert wurden, hat gezeigt, daß die Schweine gemäß Beispiel 1 bei annähernd gleichem Schlachtgewicht extrem fettarm und fleischreich waren, so daß sie den vom Markt geforderten Anforderungen besonders gut entsprachen.

## Ansprüche

1. Pharmazeutisches Mittel, enthaltend Konzentrate und/oder Konzentratfraktionen organspezifischer Peptide und/oder einzelner organspezifischer Peptide aus tierischen und/oder menschlichen Seren bzw. Organen.

2. Verwendung von Serum mit erhöhtem Gehalt an organspezifischen Peptiden zur Herstellung von Konzentraten und Konzentratfraktionen organspezifischer Peptide.

3. Verwendung von Konzentraten und/oder Konzentratfraktionen organspezifischer Peptide und/oder einzelner organspezifischer Peptide aus Schweineserum, fötalem Kälberserum, Kälberserum, Rinderserum und/oder humanem Serum bzw. tierischen und/oder menschlichen Organen in der Human-und Veterinärmedizin sowie für die Geriatrie. Verabreichungsformen: Oral (Kapseln, Tabletten), parenteral in physiologischer Kochsalzlösung und auf Salbenbasis. Verabreichung separat und/oder kombiniert mit Corticosteroiden, Schilddrüsenhormonen bzw. Fungiziden und/oder Antibiotika.

4. Verwendung von Serum mit erhöhtem Gehalt an organspezifischen Peptiden als Zusatz zu Gewebekulturen.

5. Verwendung von Schweinefutter, in dem die Kohlenhydrate zu mehr als 50 %, vorzugsweise mehr als 90 % isokalorisch ersetzt sind durch Fett zur Erzeugung von fettarmen und fleischreichen Schlachtschweinen.

6. Verfahren zur Herstellung von Serum mit einem erhöhten Gehalt an organspezifischen Peptiden, dadurch gekennzeichnet, daß die Tiere mindestens 10 Tage vor dem Schlachten gefüttert werden mit einem Futter, in dem die Kohlenhydrate zu mehr als 50 % isokalorisch ersetzt sind durch Fett.

7. Verfahren gemäß Anspruch 6, dadurch gekennzeichnet, daß die Tiere mindestens 20 Tage vor dem Schlachten gefüttert werden mit einem Futter, in dem die Kohlenhydrate zu mehr als 90 % isokalorisch ersetzt sind durch Fett.

8. Verfahren gemäß Ansprüchen 6 oder 7, dadurch gekennzeichnet, daß das Futter einen inerten Ballaststoff enthält, der im Verdauungstrakt des Tieres nicht zu Kohlenhydraten gespalten wird.

9. Verfahren gemäß Anspruch 7, dadurch gekennzeichnet, daß das Serum ab 4 Wochen nach der Futterumstellung in vierwöchigem Abstand durch Blutentzug am narkotisierten Tier gewonnen wird.

Abbildung 1

Zunahme der gesamtanabolen Aktivität des Serums
bei der Ratte durch Verfütterung der Fettdiät

Tage Fettdiät

▲——▲ Fettdiät

●——● Kontrolldiät

Abbildung  2

$E_{280nm}$

Gläschen-Nr.

Fraktion:

70 - 50

III

42-33

II

32-20

I

100   90   80   70   60   50   40   30   20   10   0

Abbildung 3

$E_{280\ nm}$

| Gläschen-Nr. 50 − 30 | Gläschen-Nr. 29 − 20 | Gläschen-Nr. 19 − 11 | |
|---|---|---|---|
| III | II | I | Fraktion |